Europäisches Patentamt

European Patent Office

Office européen des brevets

(19)

(11) Numéro de publication: **0 252 799 B1**

# (12) FASCICULE DE BREVET EUROPEEN

(45) Date de publication de fascicule du brevet: **15.04.92**   (51) Int. Cl.⁵: **C12P 19/18**, C13K 13/00

(21) Numéro de dépôt: **87401445.9**

(22) Date de dépôt: **24.06.87**

(54) **Procédé de préparation à partir du saccharose d'un mélange de sucres à haute teneur en isomaltose par voie enzymatique.**

(30) Priorité: **09.07.86 FR 8609989**

(43) Date de publication de la demande:
**13.01.88 Bulletin 88/02**

(45) Mention de la délivrance du brevet:
**15.04.92 Bulletin 92/16**

(84) Etats contractants désignés:
**AT BE CH DE ES FR GB GR IT LI LU NL SE**

(56) Documents cités:

**CHEMICAL ABSTRACTS, vol. 99, no. 19, 7 novembre 1983, page 488, résumé no. 156809a, Columbus, Ohio, US; S. FUJII et al.: "Isolation and characterization of oligosaccharides produced from sucrose by trans-glucosylation action of Serratia plymutica", & NIPPON SHOKUHIN KOGYO GAKKAISHI 1983, 30(6), 339-44**

**CARBOHYDRATE RESEARCH, vol. 108, 1982, pages 279-283, Elsevier Scientific Publishing Co., Amsterdam, NL; K.L. SMILEY et al.: "A simplified method for preparing linear isomalto-oligosaccharides"**

(73) Titulaire: **Socété Anonyme dite: AGRO INDUS-TRIE RECHERCHES ET DEVELOPPEMENTS 27-29 rue Chateaubriand F-75383 Paris(FR)**

(72) Inventeur: **Paul, Francois Bernard
28, rue du Vivier
F-31650 Saint -Orens de Gameville(FR)**
Inventeur: **Monsan, Pierre Frédéric
Renoufail Mondonville
F-31700 Blagnac(FR)**
Inventeur: **Remaud, Magali Martine Claude
80, rue Fontaine des Cerdans
F-31520 Ramonville(FR)**
Inventeur: **Pelenc, Vincent Pascal
25, Avenue Jules Julien
F-31400 Toulouse(FR)**

(74) Mandataire: **Colas, Jean-Pierre et al
Cabinet de Boisse 37, avenue Franklin D. Roosevelt
F-75008 Paris(FR)**

EP 0 252 799 B1

## Description

L'invention concerne un procédé de préparation par voie enzymatique d'un sirop à haute teneur en isomaltose, ainsi que le sirop obtenu.

L'isomaltose est un produit intermédiaire de valeur pour la préparation par hydrogénation catalytique, de l'isomaltitol (ou D-sorbitol-6-O-$\alpha$-D-glucopyranosyle). L'isomaltitol présente un pouvoir sucrant important, est faiblement calorique, n'est pas cariogénique et peut donc constituer un substitut intéressant du sucre.

Egalement, l'isomaltose, qui constitue un dimère du glucose, présente une liaison glucosidique (1 → 6) qui est relativement stable et est un substrat potentiel pour des transestérifications dans le but de produire des graisses faiblement caloriques, par exemple.

Jusqu'à présent, l'isomaltose a été préparé par traitement chimique ou enzymatique de divers substrats ou par hydrolyse chimique ou enzymatique du dextrane produit par la bactérie lactique Leuconostoc mesenteroides NRRL B512F, suivi d'étapes de fractionnement des solutions obtenues en faisant passer celles-ci sur des colonnes de résines cationiques fortement acides de grande hauteur (10-15 mètres) à température relativement élevée (45-85°C) par exemple corne décrit dans US-A-4 521 252. On obtient ainsi des sirops dont l'extrait sec contient plus de 40% d'isomaltose, que l'on peut encore purifier davantage au prix d'opérations supplémentaires. Ces procédés sont compliqués et exigent un appareillage de purification important pour leur mise en oeuvre.

La présente invention vise à fournir un procédé de synthèse simple d'un sirop à haute teneur en isomaltose à partir de saccharose par traitement de ce dernier à l'aide d'une combinaison originale de deux enzymes.

Plus particulièrement, l'invention concerne un procédé de préparation d'un sirop dont l'extrait sec contient une teneur en isomaltose d'au moins 39% en poids, caractérisé en ce qu'il consiste à traiter une solution aqueuse de saccharose par un mélange des enzymes dextrane-saccharase (EC : 2.4.1.5.) et dextranase (EC : 3.2.1.11 et 3.2.1.94) à une température comprise entre 0 et 50°C et à un pH dans la gamme de 4,5 à 7, de façon à obtenir un sirop aqueux comprenant de l'isomaltose, du fructose, du glucose et, éventuellement, de l'isomaltotriose comme constituants principaux.

La réaction enzymatique peut être conduite de façon discontinue (par charges) ou continue (réacteur à enzymes immobilisées). On peut indifféremment utiliser des enzymes libres ou immobilisées ou l'une des deux enzymes seulement peut être immobilisée. Une conversion complète du saccharose peut être obtenue avec une durée de réaction suffisante. Celle-ci sera d'autant plus courte que l'on utilisera une quantité d'enzymes plus importante et une température de réaction plus élevée. A titre indicatif, on peut utiliser des durées de réaction comprises entre 1 et 200 heures environ, de préférence de 20 à 80 heures, pour un mode de mise en oeuvre discontinu.

La réaction peut être décrite comme suit : la dextrane-saccharase polymérise le résidu glucosyle du saccharose et libère le fructose dans le milieu réactionnel. La dextranase qui produit spécifiquement de l'isomaltose empêche la formation du polymère (dextrane) en hydrolysant la liaison glucosidique $\alpha$1→6 au fur et à mesure de sa formation par la dextrane-saccharase. Le milieu s'enrichit donc en isomaltose au fur et à mesure de la conversion du saccharose. L'action simultanée de ces deux enzymes permet d'éviter la formation de dextranes hautement polymérisés présentant des ramifications $\alpha$1→3 en particulier qui diminuent notablement le rendement d'hydrolyse du dextrane par la dextranase.

La réaction décrite dans ce procédé permet d'éviter totalement la production d'oligodextranes ramifiés qui constituent jusqu'à 20% des produits d'hydrolyse enzymatique du dextrane. De l'isomaltotriose peut ensuite se former, selon les conditions, par transfert du glucose sur l'isomaltose par la dextrane-saccharase, en même temps que des quantités mineures d'autres ingrédients tels que des oligosaccharides de degré de polymérisation allant de 4 à 6, et du leucrose.

Un autre avantage très important du procédé décrit est que le substrat de départ de la réaction est le saccharose, bon marché et disponible en grande quantité comparativement au dextrane, produit relativement cher même peu purifié.

La quantité de fructose produite représente environ la moitié du saccharose de départ et constitue un produit intéressant qui peut être valorisé à part.

Avantageusement, donc, le procédé comprend l'étape supplémentaire consistant à séparer de la solution aqueuse obtenue, la majeure partie du fructose. Cette séparation peut s'effectuer commodément en faisant passer la solution aqueuse dans une colonne garnie de résine échangeuse de cations sous la forme Ca$^{++}$, en exploitant la propriété, connue en soi, de l'affinité d'une telle résine pour le fructose.

La température du traitement enzymatique est comprise entre 0 et 50°C, de préférence entre 15 et 35°C et tout particulièrement entre 25 et 30°C pour favoriser la production d'isomaltose.

Le pH doit être maintenu dans la gamme de 4,5 à 7, par exemple en utilisant un tampon approprié. On préfère opérer à un pH de 5 à 6.

Les concentrations des enzymes ne sont pas non plus étroitement critiques. Il faut au moins 0,1 U/ml de chaque enzyme pour obtenir un effet notable. Il n'y a pas d'inconvénient à utiliser de grandes quantités d'enzymes si ce n'est que des quantités excessivement grandes sont anti-économiques. A titre indicatif des concentrations de 0,2 à 2 U/ml environ de dextrane-saccharase et de 0,2 à 5 U/ml environ de dextranase se sont révélées généralement satisfaisantes.

Quant à la concentration en substrat (saccharose) elle peut aller de 10 à 300 g/litre, de préférence de 50 à 200 g/litre. En-dessous de 10 g/l, il y a insuffisance de substrat. Au-dessus de 300 g/l, la formation de leucrose devient importante. Le leucrose est un disaccharide produit par la dextrane-saccharase qui transfère le résidu glucosyle, provenant du saccharose sur le fructose libre présent dans le milieu réactionnel. Ce disaccharide est un contaminant indésirable. De plus, la proportion d'isomaltotriose s'accroît notablement au-dessus de 200 g/l.

L'invention concerne aussi le sirop aqueux d'isomaltose obtenu par le procédé de l'invention ainsi que tout extrait sec obtenu par un processus de séchage adéquat (lyophilisation, atomisation, etc...).

Les exemples non limitatifs suivants sont donnés en vue d'illustrer l'invention.


Exemples 1 à 12

Toutes les synthèses enzymatiques ont été réalisées dans du tampon acétate de sodium 20 millimolaire de pH 5,2, à une température de 20°C, et pendant une durée de réaction de 48 heures environ. Dans ces conditions, la conversion du saccharose est complète.

On a utilisé de la dextrane saccharase de Leuconostoc mesenteroides NRRL B512F, provenant de la Société Bio Europe, et de l'endodextranase D1508 provenant de la Société SIGMA.

La solution obtenue a été purifiée (séparation de la majeure partie du fructose) en la faisant passer sur une colonne (hauteur : 1 m, diamètre : 5 cm, volume utile : 2 litres) garnie de résine échangeuse de cations sous forme $Ca^{++}$ de marque déposée Duolite C204F, provenant de la Société Duolite, à la température ambiante. Le fructose retenu par la colonne peut être isolé et valorisé à part.

Après cette purification, le sirop obtenu a été analysé par chromatographie en phase liquide de haute peformance (HPLC) sur une colonne $\mu$ Bondapack C18 Millipore Waters, l'éluant utilisé étant de l'eau ultra-pure.

Les conditions opératoires de chaque exemple et les résultats obtenus sont consignés dans le tableau suivant :

| EXEMPLES | 1 | 2 | 3 | 4 | 5 | 6 | 7 | 8 | 9 | 10 | 11 | 12 |
|---|---|---|---|---|---|---|---|---|---|---|---|---|
| CONCENTRATION DE SACCHAROSE g/litre | 50 | 50 | 50 | 50 | 100 | 100 | 100 | 100 | 200 | 200 | 200 | 200 |
| DEXTRANE SACCHARASE, Unités/ml | 0,5 | 0,5 | 0,5 | 0,5 | 0,5 | 1 | 1 | 1 | 0,5 | 1 | 1 | 1 |
| DEXTRANASE, Unités/ml | 0,5 | 1 | 2 | 4,8 | 0,5 | 1 | 2 | 4 | 0,5 | 1 | 2 | 4 |
| % D'ISOMALTOSE DANS L'EXTRAIT SEC | 63 | 67 | 40 | 52 | 62 | 56 | 60 | 64 | 60 | 39 | 53 | 55 |
| % D'ISOMALTOTRIOSE DANS L'EXTRAIT SEC | 16,6 | 6 | traces | 3 | 30 | 33,7 | 29 | 24 | 35,4 | 30,1 | 33,9 | 33,6 |

On voit, d'après les résultats obtenus, que, lorsque la concentration du substrat saccharose est accrue pour des concentrations similaires des deux enzymes, on observe une chute du rendement en isomaltose et une augmentation du rendement en isomaltotriose.

La proportion de glucose présente dans le produit final varie en fonction des conditions opératoires. Bien que n'ayant pas été mesurée directement, elle représente l'essentiel de la différence entre 100% et la somme des pourcentages indiquée pour l'isomaltose et l'isomaltotriose, les autres constituants éventuellement présents ne l'étant qu'en très petites quantités. En ce qui concerne la teneur en fructose résiduel, on peut l'estimer à 2-3% en poids de l'extrait sec.

Exemples 13 à 15

Une deuxième série d'essais a été réalisée en utilisant une température de réaction de 30°C, les autres conditions étant semblables à celles décrites pour les exemples 1-12.

Le tableau suivant résume les données et résultats de ces essais.

| EXEMPLES | 13 | 14 | 15 |
|---|---|---|---|
| SACCHAROSE, g/l | 50 | 100 | 200 |
| DEXTRANE SACCHARASE U/ml | 1 | 1 | 1 |
| DEXTRANASE, U/ml | 1 | 1 | 1 |
| % D'ISOMALTOSE | 80 | 70 | 65 |
| % D'ISOMALTOTRIOSE | 0 | 12 | 31 |
| GLUCOSE, LEUCROSE OLIGOSACCHARIDES (traces) | 20 | 18 | 4 |

On voit d'après ces résultats que l'élévation de température a 30°C a un effet bénéfique, spécialement pour les concentrations en saccharose relativement faibles. Ceci probablement en raison d'une hydrolyse plus importante de l'isomaltotriose.

Il va de soi que les modes de réalisation décrits ne sont que des exemples et qu'on pourrait les modifier, notamment par substitution d'équivalents techniques, sans sortir pour cela du cadre de l'invention.

**Revendications**

1. Procédé de préparation d'un sirop dont l'extrait sec contient une teneur en isomaltose d'au moins 39% en poids, caractérisé en ce qu'il consiste à traiter une solution aqueuse de saccharose par un mélange des enzymes dextrane-saccharase et dextranase à une température comprise entre 0 et 50°C et à un pH dans la gamme de 4,5 à 7, de façon à obtenir un sirop aqueux comprenant de l'isomaltose, du fructose, du glucose et, éventuellement, de l'isomaltotriose comme constituants principaux.

2. Un procédé selon la revendication 1, caractérisé en ce qu'il comprend l'étape supplémentaire consistant à séparer de la solution aqueuse la quasi totalité du fructose.

3. Un procédé selon la revendication 1, caractérisé en ce que la température de la réaction enzymatique est comprise dans la gamme de 15 à 35°C environ.

4. Un procédé selon la revendication 3, caractérisé en ce que la température de la réaction enzymatique est comprise dans la gamme de 25 à 30°C.

5. Un procédé selon la revendication 1, 3 ou 4, caractérisé en ce que la durée de la réaction enzymatique est comprise entre 1 et 200 heures.

6. Un procédé selon la revendication 5, caractérisé en ce que la durée de la réaction enzymatique est comprise entre 20 et 80 heures environ.

7. Un procédé selon l'une quelconque des revendications 1 et 3-6, caractérisé en ce que la réaction enzymatique est conduite à un pH de 5 à 6.

8. Un procédé selon l'une quelconque des revendications 1 et 3-7, caractérisé en ce qu'on utilise de 0,2 à 2 U/ml de dextrane-saccharase et de 0,2 à 5 U/ml de dextranase.

9. Un procédé selon l'une quelconque des revendications 1 et 3-8, caractérisé en ce que la concentration du saccharose dans la solution aqueuse de départ est comprise entre 10 et 300 g/litre.

10. Un procédé selon la revendication 9, caractérisé en ce que ladite concentration est comprise dans la gamme de 50 à 200 g/litre.

**Claims**

1.  Process for preparing a syrup of which the dry extract has an isomaltose content of at least 39% by weight, characterized by the fact that it consists of the treatment of an aqueous solution of sucrose with a mixture of dextrane-saccharase and dextranase enzymes at a temperature between 0 and 50° and a pH value in the range 4.5 to 7, in such a way as to obtain an aqueous syrup comprising isomaltose, fructose, glucose and possibly isomaltotriose as the main constituents.

2.  A process in accordance with Claim 1, characterized by the fact that it comprises the supplementary stage consisting of the separation of almost all the fructose from the aqueous solution.

3.  A process in accordance with Claim 1, characterized by the fact that the fact that the temperature of the enzymatic reaction is included in the range of about 15-35°C.

4.  A process in accordance with Claim 3, characterized by the fact that the temperature of the enzymatic reaction is included in the range 25-30°C.

5.  A process in accordance with Claim 1,3 or 4, characterized by the fact that the duration of the enzymatic reaction is between 1 and 200 hours.

6.  A process in accordance with Claim 5, characterized by the fact that the duration of the enzymatic reaction is approximately between 20 and 80 hours.

7.  A process in accordance with any one of Claims 1 and 3-6, characterized by the fact that the enzymatic raction is effected at a pH value of 5-6.

8.  A process in accordance with any one of Claims 1 and 3-7,characterized by the fact that between 0.2 and 2 U/ml of dextrane-saccharose and between 0.2 and 5 U/ml of dextranase are used.

9.  A process in accordance with any one of Claims 1 and 3-8 ,characterized by the fact that the concentration of saccharose in the aqueous starting solution ranges between 10 and 300 g/litres.

10. A process in accordance with Claim 9,characterized by the fact that the said concentration ranges from 50 to 200 g/litre.

**Patentansprüche**

1.  Verfahren zur Herstellung eines Sirups, dessen Trockenextrakt einen Gehalt an Isomaltose von wenigstens 39 Gew.-% aufweist, dadurch gekennzeichnet, daß es darin besteht, eine wäßrige Saccharose-Lösung mit einer Mischung der Enzyme Dextransaccharase und Dextranase bei einer Temperatur zwischen 0 und 50°C und einem pH im Bereich von 4,5 bis 7 in einer Weise zu behandeln, daß ein wäßriger Sirup erhalten wird, der als Hauptbestandteile Isomaltose, Fructose, Glucose und gegebenenfalls Isomaltotriose enthält.

2.  Verfahren nach Anspruch 1, dadurch gekennzeichnet, daß es einen zusätzlichen Schritt umfaßt, der darin besteht, nahezu die gesamte Fructose aus der wäßrigen Lösung abzutrennen.

3.  Verfahren nach Anspruch 1, dadurch gekennzeichnet, daß die Temperatur der enzymatischen Reaktion im Bereich von etwa 15 bis 35°C liegt.

4.  Verfahren nach Anspruch 3, dadurch gekennzeichnet, daß die Temperatur der enzymatischen Reaktion im Bereich von 25 bis 30°C liegt.

5.  Verfahren nach Anspruch 1, 3 oder 4, dadurch gekennzeichnet, daß die Dauer der enzymatischen Reaktion zwischen 1 und 200 h liegt.

6. Verfahren nach Anspruch 5, dadurch gekennzeichnet, daß die Dauer der enzymatischen Reaktion zwischen etwa 20 und 80 h liegt.

7. Verfahren nach irgendeinem der Ansprüche 1 und 3 bis 6, dadurch gekennzeichnet, daß die enzymatische Reaktion bei einem pH von 5 bis 6 durchgeführt wird.

8. Verfahren nach irgendeinem der Ansprüche 1 und 3 bis 7, dadurch gekennzeichnet, daß 0,2 bis 2 E/ml Dextransaccharase und 0,2 bis 5 E/ml Dextranase eingesetzt werden.

9. Verfahren nach irgendeinem der Ansprüche 1 und 3 bis 8, dadurch gekennzeichnet, daß die Saccharose-Konzentration in der wäßrigen Ausgangslösung zwischen 10 und 300 g/l liegt.

10. Verfahren nach Anspruch 9, dadurch gekennzeichnet, daß diese Konzentration im Bereich von 50 bis 200 g/l liegt.